# EUROPEAN PATENT SPECIFICATION

(11) **EP 2 134 838 B1**
(45) Date of publication and mention of the grant of the patent: **03.04.2013**
(21) Application number: 08717761.4
(22) Date of filing: 13.03.2008
(51) Int. Cl.: C12N 9/64

(54) **CONCENTRATE OF PROTEIN C CONTAINING PROTEIN S, ITS PREPARATION AND USE**
PROTEIN-C-KONZENTRAT MIT PROTEIN S SOWIE HERSTELLUNG UND VERWENDUNG DAVON
CONCENTRÉ DE PROTÉINE C CONTENANT LA PROTÉINE S, SA PRÉPARATION ET SON UTILISATION

(30) Priority: 15.03.2007 IT FI20070063
(43) Date of publication of application: 23.12.2009
(73) Proprietor: Kedrion S.P.A., 55051 Castelvecchio Pascoli - Barga (IT)
(72) Inventor: ANGELINI, Cristina, I-55031 Camporgiano (IT); FARINA, Claudio, I-56122 Pisa (IT); NARDINI, Claudia, I-55100 Lucca (IT); FRANCESCHINI, Rodolfo, I-56122 Pisa (IT)
(74) Representative: Gervasi, Gemma
(86) International application number: PCT/EP2008/053017
(87) International publication number: WO 2008/110603

(56) References cited:
- WO-A-2006/044294
- US-A- 5 093 117
- DIEHL J L ET AL: "Sepsis and coagulation." CURRENT OPINION IN CRITICAL CARE OCT 2005, vol. 11, no. 5, October 2005 (2005-10), pages 454-460, XP009100635 ISSN: 1070-5295
- RADOSEVICH M ET AL: "Chromatographic purification and properties of a therapeutic human protein C concentrate" JOURNAL OF CHROMATOGRAPHY B: BIOMEDICAL SCIENCES & APPLICATIONS, ELSEVIER, AMSTERDAM, NL, vol. 790, no. 1-2, 25 June 2003 (2003-06-25), pages 199-207, XP004426800 ISSN: 1570-0232
- BURNOUF T ET AL: "Application of bioaffinity technology in therapeutic extracorporeal plasmapheresis and large-scale fractionation of human plasma" JOURNAL OF CHROMATOGRAPHY B: BIOMEDICAL SCIENCES & APPLICATIONS, ELSEVIER, AMSTERDAM, NL, vol. 715, no. 1, 11 September 1998 (1998-09-11), pages 65-80, XP004146984 ISSN: 1570-0232
- YAN S C B ET AL: "CHARACTERIZATION AND NOVEL PURIFICATION OF RECOMBINANT HUMAN PROTEIN C FROM THREE MAMMALIAN CELL LINES" BIO/TECHNOLOGY, NATURE PUBLISHING CO. NEW YORK, US, vol. 8, no. 7, 1 July 1990 (1990-07-01), pages 655-661, XP008028568 ISSN: 0733-222X
- RIGBY ALAN C ET AL: "PROTEIN S: A CONDUIT BETWEEN ANTICOAGULATION AND INFLAMMATION" CRITICAL CARE MEDICINE, WILLIAMS AND WILKINGS COMPANY, BALTIMORE, MA, US, vol. 32, no. 5 SUPPL, 1 May 2004 (2004-05-01), pages S336-S341, XP009076163 ISSN: 0090-3493

## Description

### Field of the invention

The invention relates to the field of purification of human plasma proteins and their use.

### State of the art

Protein C is the zymogen of a vitamin K-dependent serine protease which, together with its cofactor Protein S, performs a fundamental role in regulating hemostasis both under physiological conditions and if the system is altered due to a pathological condition. Protein C activation takes place by means of the thrombin-thrombomodulin complex and is accelerated approximately 20-fold when Protein C is complexed with EPCR (endothelial cell Protein C receptor). Activated Protein C binds to Protein S then proceeds to exert its proteolytic effect on factors Va and VIIIa, as well as on their respective inactive precursors, thus exerting its anticoagulation function. Activated Protein C also has a profibrinolytic action since it inactivates PAI-1 and TAFI (thrombin-activatable fibrinolysis inhibitor) and an anti-inflammatory action since, when complexed with the EPCR receptor, it inhibits release of inflammatory cytokines (tumor necrosis factor, interleukin 6) from monocytes by inhibiting a nuclear transcriptase (NF-kB) involved in the release of said cytokines and the migration and adhesion of neutrophils (Esmon T. et al. "The Protein C Pathway" Crit Care Med 28, No. 9 (Suppl.), S44-S48 (2000); Esmon T et al. "Protein C anticoagulant pathway and its role in controlling microvascular thrombosis and inflammation" Crit Care Med 29, No. 7 (Suppl.), S48-S51 (2001)).

Protein S performs an anticoagulant action as a cofactor of activated Protein C and also has an independent anticoagulant action due to inhibition of the prothrombinase complex (Factor Xa, Factor Va and Prothrombin) and the tenase complex (Factor IXa, Factor VIIIa, calcium ions and Factor X). When complexed with C4BP, Protein S also exhibits anti-inflammatory action which is brought about by localizing the Protein S-C4 binding protein complex to the surface of apoptotic cells expressing phosphatidylserine, thus facilitating clearance of these cells by macrophages before their rupture and consequent release of potent proinflammatory intracellular molecules, and increasing binding of C4BP at neutrophil surfaces in order to regulate proliferation of complement at sites of coagulation system activation (Rigby A.C. and al. "Protein S: A conduit between anticoagulation and inflammation" Crit Care Med 32, No. 5, S336-S341 (2004); Webb JH and al. "Vitamin K-dependent Protein S localizing complement regulator C4b-binding protein to the surface of apoptotic cells" J Immunol.169, 2580-2586 (2002); Anderson HA et al. "Serum-derived Protein S binds to phosphatidylserine and stimulates the phagocytosis of apoptotic cells" Nat. Immunol. 4, 87-91 (2003); Rezende S.M. and al. "Coagulation, inflammation, and apoptosis: different roles for Protein S and the Protein S-C4b binding Protein Complex" Blood 13, No. 4, 1192-1201, (2004)).

Sepsis is a complex pathological condition characterized by a systemic inflammatory reaction which can be triggered by bacterial, fungal or viral infections, by multiple traumas, serious burns or organ transplants of which prognosis is often fatal. The hemostatic and fibrinolytic systems are closely involved in the pathophysiology of sepsis. In sepsis, inflammation and coagulation are mutually amplifying leading to ischemia of the microcirculation and organ malfunction. The septic patient shows altered blood levels of several coagulation factors, in particular a reduction in Protein C and Protein S levels, with the establishment of a consequent hypercoagulability state due to the capacity of this anticoagulation pathway for regulating prothrombin production being compromised. Variations in blood levels of these factors are an indicator of thrombotic lesion severity and are correlated with prognosis, there being an inversely proportional correlation between plasma levels and mortality (Fourrier F. et al. "Septic shock, multiple organ failure, and disseminated intravascular coagulation: Compared patterns of antithrombin III, Protein C, and Protein S deficiences" Chest 101, 816-823 (1992); Fisher CJ Jr "Protein C levels as a prognostic indicator of outcome in sepsis and related diseases" Cri. Care Med. 28 (9 Suppl), S49-S56 (2000); La Rosa SP et al. "Decreased Protein C, Protein S, and antithrombin levels are predictive of poor outcome in Gram-negative sepsis caused by Burkholderia pseudomallei" Int J Infect Dis.1 0 (1), 25-31 (2006)). Activated Protein C is the only drug currently authorized for use in sepsis and is active at paraphysiological concentrations. However, this drug presents a number of contraindications due to the high hemorrhagic risk associated therewith, which excludes its use in many septic patients and in pediatric patients. Furthermore, in a recent randomized double-blind placebo-controlled study, Protein C activation was also demonstrated in meningococcal sepsis in which a reduced activation had been assumed, an opinion derived from the hypothesis that inflammatory cytokines produced during sepsis modulate thrombomodulin and EPCR downregulation (De Kleijn ED et al. "Activation of Protein C following infusion of Protein C concentrate in children with severe meningococcal sepsis and purpura fulminans: a randomized, double-blinded, placebo-controlled, dose-finding study" Crit Care Med. 31 (6), 1839-47 (2003)).

A number of clinical trials conducted using a Protein C concentrate in meningococcemia have demonstrated a significantly reduced morbidity and mortality in the various populations studied. In all the reported cases an increase in plasma levels of activated Protein C, as well as a tendency to normalize related coagulation parameters and an improvement in the final outcome were recorded. Neither adverse reactions nor hemorrhagic complications were recorded, especially in pediatric patients (White B et al. "An open-label study of the role of adjuvant hemostatic support with Protein C replacement therapy in purpura fulminans-associated meningococcemia" Blood 96, 3719-3724 (2000); De Kleijn ED et al. "Activation of Protein C following infusion of Protein C concentrate in children with severe meningococcal sepsis and purpura fulminans: a randomized, double-blinded, placebo-controlled, dose-finding study" Crit Care Med. 31(6), 1839-47 (2003); Baratto et al. "Use of Protein C concentrate in adult patients with severe sepsis and septic shock" Minerva Anestesiol 70, 351-6 (2004); Pettenazzo et al. "Use of Protein C concentrate in critical conditions: clinical experience in pediatric patients with sepsis" Minerva Anestesiol. 70(5), 357-63 (2004); Silvani P et al. "Use of Protein C in pediatric patients with sepsis" Minerva Anestesiol. 71, 373-78 (2005); Schellongowski P. et al. "Treatment of adult patients with sepsis-induced coagulopathy and purpura fulminans using a plasma-derived Protein C concentrate (Ceprotin)" Vox Sanguinis 90, 294-301 (2006); etc). Administration of a Protein C concentrate containing Protein S has not yet been the subject of any clinical trials, despite Protein S presence in the concentrate providing additional protection against thrombotic events. Furthermore it is reasonable to assume a synergistic effect between the two proteins in at least partly correcting any hemostatic system imbalances and improving the general clinical situation by reducing proinflammatory cytokine release, and hence to assume the possible therapeutic application of a concentrate of Protein C and Protein S in sepsis, particularly in cases where activated recombinant Protein C administration is strongly contraindicated due to its high hemorrhagic risk, as is the case with pediatric patients (RESOLVE trial).

In addition to the absence of hemorrhagic risks, a Protein C concentrate containing Protein S presents a further advantage compared to activated recombinant Protein C, namely the decidedly lower cost of the product and hence of the complete treatment per patient.

Radosevich M. et al. (J. Chromatography B: Biomed. Sc.&Appl. 2003, 790, 199-207) discloses a process for the preparation of a concentrate of protein C from cryo-poor plasma by means of four-steps chromatographic procedure. The method described make use of a waste fraction of the purification of factor IX from frozen human plasma. The last chromatographic step is performed by affinity on heparin-sephasrose CL6B. The product obtained by this procedure is enriched in protein C (but it contains less than 0.01 Ul/ml of protein S).

A product consisting of human Protein C is commercially available in which, however, Protein S is absent.

### Summary of the invention

The invention relates to a process for obtaining a concentrate of Protein C also comprising Protein S wherein :
a) the fraction collected after the second wash of DEAE-Sepharose resin used for obtaining the purified Prothrombin Complex (PTC) starting from frozen human plasma is dialyzed with a suitable buffer and concentrated;
b) the product derived from the previous step is subjected to chromatography on weak anion-exchange resin and the pH of the eluate containing the proteins of interest is adjusted followed by dialysis with the conditioning buffer of the resin used in the second chromatographic step;
c) a second chromatographic step is carried out on a resin which selectively binds Protein C and Protein S, wherein said resin is hydroxyapatite;
d) the purified protein mixture obtained in this way is dialyzed with a suitable formulation buffer, then prefiltered, nanofiltered and lyophilised.

### Brief description of the figure

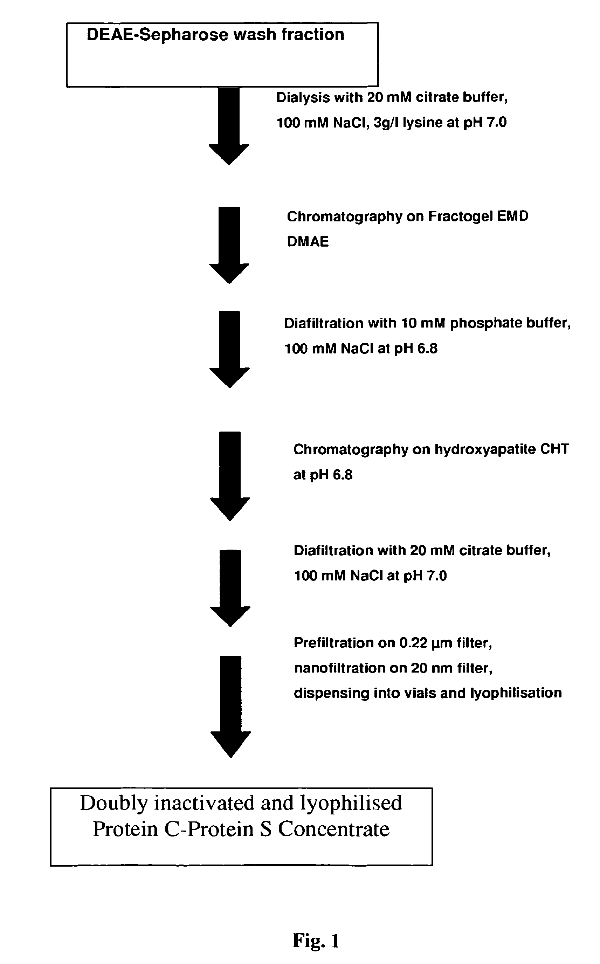

Figure 1 shows the various steps of the process of the invention in schematic form.

### Detailed description of the invention

The invention relates to a method for purifying, from human plasma, a concentrate of Protein C containing Protein S, and the product obtained in this manner.

The starting material for this new purification method is the fraction collected from the second washing of the DEAE-Sepharose resin used for obtaining purified Prothrombin Complex (PTC) from frozen human plasma by means of a known process. This fraction is currently a waste fraction of said production process. The wash fraction used as the starting material, having already previously undergone a viral inactivation step by solvent/detergent (S/D) treatment is then dialyzed with a suitable buffer and subjected to chromatography with a weak anion-exchange resin; the proteins of interest which remain fixed to the resin are then eluted with a buffer of higher ionic strength.

The eluate containing the proteins of interest is diafiltered using the conditioning buffer of the resin utilized in the second chromatographic step at a pH close to neutrality.

The second chromatographic step uses a resin able to selectively bind proteins C and S and, after eluting with a suitable high ionic strength buffer, provides a purified concentrate of the two proteins of interest.

The purified protein mixture obtained in this manner is diafiltered with a suitable formulation buffer, then prefiltered, nanofiltered and lyophilised.

The lyophilisate thus obtained is stored at 4°C.

The final product obtained in this manner is subjected to two viral inactivation/reduction processes (solvent/detergent treatment and nanofiltration) which ensure the removal of both enveloped and non-enveloped viruses, thus enabling the current safety profiles for viruses to be fully satisfied.

The lyophilised product, to be reconstituted with WFI, is intended for parenteral administration, preferably intravenous. The dose of Protein C to be administered is very variable from patient to patient because the dosage must be such as to immediately restore in the patient higher levels of Protein C than normal and then to maintain said values within the normal range by means of continuous monitoring.

A possible dosage for sepsis treatment is 50-500 IU/kg/day. In accordance with the invention the wash solution utilized as the starting material is dialized by using a citrate buffer, preferably 20 mM citrate containing 100 mM NaCl at pH 7.

The resin used for the first chromatographic step is a weak anion-exchange resin, preferably Fractogel EMD DMAE. The buffer used for eluting protein C and Protein S is a buffer at acid pH, preferably citrate containing 200 mM NaCl.

The resin used for the second chromatographic step is hydroxyapatite which is a resin able to selectively bind Proteins C and S, ; the relative conditioning buffer is a phosphate buffer, preferably 10 mM phosphate containing 100 mM NaCl at pH 6.8. The proteins of interest are eluted with phosphate buffer at the same pH and at higher NaCl concentrations, preferably 300 mM.

Finally the formulation buffer used for the dialysis after the second chromatography consists of citrate, preferably 20 mM citrate containing 100 mM NaCl and 3 g/l lysine at pH 7.

The product obtained consists of a solution containing 50 U/ml ± 10 of Protein C, > 5 U/ml of Protein S, < 0.4 U/ml of factor II, < 0.5 U/ml of factor X and < 0.05 U/ml of factor IX.

### Example 1

2250 ml of the fraction collected after the second wash of the DEAE-Sepharose resin used for obtaining purified prothrombin complex (PTC) from frozen human plasma, are dialysed with 20 mM citrate buffer, 100 mM NaCl at pH 7 ± 0.2 and concentrated about 15-fold. Chromatography is then carried out on anion-exchange resin (45 ml Fractogel EMD DMAE), eluting the proteins of interest with 20 mM citrate buffer, 200 mM NaCl at pH 5 ± 0.2. The pH of the eluate is adjusted to 7 ± 0.2, and dialysis is carried out with 10 mM phosphate buffer, 100 mM NaCl at pH 6.8 ± 0.2 followed by chromatography on a column containing 13 ml of CHT ceramic hydroxyapatite Type I. The elution in the second chromatographic step is performed with 10 mM phosphate buffer, 300 mM NaCl at pH 6.8 ± 0.2. When complete, the purified protein mixture obtained in this manner is dialysed with the formulation buffer (20 mM citrate, 100 mM NaCl at pH 7 ± 0.2) and concentrated if necessary to obtain a solution having a Protein C concentration between 40 and 60 U/ml, then prefiltered through a 0.22 µm filter, nanofiltered through a 20 nm filter, dispensed into vials and lyophilised. The volume of solution dispensed into the various vials is 10 ml, to obtain about 500 ± 100 units of Protein C per vial. The lyophilisate thus obtained is stored at 4°C.

**Scheme summarizing the course of the purification process**

| Purification process | Volume (ml) | Single step % recovery of Protein C | % Recovery of Protein C compared to initial amount | Specific activity (IU/mg) |
|---|---|---|---|---|
| Starting material (DEAE-Sepharose wash fraction) | 2250 | | | |
| Diafiltration | 160 | 100 | 100 | 3.25 |
| Chromatography on | 171 | 76.9 | 76.9 | 28.5 |
| Fractogel DMAE | | | | |
| Chromatography on CHT hydroxyapatite | 50 | 58.1 | 41.2 | 204.2 |
| Dialysis and nanofiltration | 56 | 72.8 | 30.0 | 202.5 |
| Lyophilisation | 56 | 90.6 | | 186.7 |

**Summary table of final product characteristics**

| Parameter | Analysis method | Specification |
|---|---|---|
| Total U of Protein C | Chromogenic assay | 50 U / ml ± 10 |
| Specific activity of Protein C | Chromogenic assay | 180 U / mg |
| Total U of Protein S | Elisa assay | > 5 U / ml |
| Total U of Prothrombin | Chromogenic assay | < 0.4U/ml |
| Total U of Factor X | Elisa assay | < 0.5 U / ml |
| Total U of Factor IX | Coagulation assay | <0.05U/ml |

## Claims

1. Process for obtaining a concentrate of Protein C also comprising Protein S wherein :
a) the fraction collected after the second wash of DEAE-Sepharose resin used for obtaining the purified Prothrombin Complex (PTC) starting from frozen human plasma is dialyzed with a suitable buffer and concentrated;
b) the product derived from the previous step is subjected to chromatography on weak anion-exchange resin and the pH of the eluate containing the proteins of interest is adjusted followed by dialysis with the conditioning buffer of the resin used in the second chromatographic step;
c) a second chromatographic step is carried out on a resin which selectively binds Protein C and Protein S, wherein said resin is hydroxyapatite;
d) the purified protein mixture obtained in this way is dialyzed with a suitable formulation buffer, then prefiltered, nanofiltered and lyophilised.

2. Concentrate of Protein C also comprising Protein S obtained by the process of claim 1.

3. Concentrate according to claim 2 wherein the content of Protein S is equal to at least 10% (calculated in U/ml) of the content of Protein C.

4. Concentrate according to claim 3 wherein the content of Factor II, Factor X and Factor IX is < 0.5 U/ml.

5. Concentrate according to claim 4 wherein the content of Protein C is 40-60 U/ml, the content of Protein S is > 5 U/ml and the content of Factor II, Factor X and Factor IX is < 0.5 U/ml.

6. Use of the concentrate according to claims 2-5 for the preparation of pharmaceutical compositions for the treatment of sepsis as well as for congenital Protein C and Protein S deficiencies.

7. Use according to claim 6 wherein said formulations are in a form for parenteral administration.

8. Use according to claim 6 wherein said formulation consists of a lyophilisate of the solution containing Protein C and Protein S, and WFI for the reconstitution thereof.

## Patentansprüche

1. Verfahren zum Erhalten eines Konzentrats von Protein C, das auch Protein S umfaßt, worin
(a) die Fraktion, die nach der zweiten Waschung eines DEAE-Sepharose-Harzes gewonnen wurde, das für ein Erhalten des gereinigten ProthrombinKomplexes (purified Prothrombin Complex; PTC) ausgehend von gefrorenem Humanplasma verwendet wurde, mit einem geeigneten Puffer dialysiert und konzentriert wird;
(b) das von dem vorangehenden Schritt abgeleitete Produkt einer Chromatographie an einem schwachen Anion-Austausch-Harz unterworfen wird und der pH-Wert des die Proteine von Interesse enthaltenden Eluats eingestellt wird, gefolgt von einer Dialyse mit dem Konditionierungs-Puffer des in dem zweiten Chromatographie-Schritt verwendeten Harzes;
(c) ein zweiter Chromatographie-Schritt an einem Harz durchgeführt wird, das selektiv Protein C und Protein S bindet, wobei das Harz Hydroxyapatit ist;
(d) die auf diesem Weg erhaltene gereinigte Protein-Mischung mit einem geeigneten Formulierungs-Puffer dialysiert wird, danach vorfiltiert wird, nanofiltriert wird und lyophilisiert wird.

2. Konzentrat von Protein C, auch umfassend Protein S, erhalten nach dem Verfahren von Anspruch 1.

3. Konzentrat nach Anspruch 2, worin der Gehalt an Protein S gleich wenigstens 10 % (berechnet in U/ml) des Gehalts an Protein C ist.

4. Konzentrat nach Anspruch 3, worin der Gehalt an Faktor II, Faktor X und Faktor IX < 0,5 U/ml ist.

5. Konzentrat nach Anspruch 4, worin der Gehalt an Protein C 40 bis 60 U/ml ist, der Gehalt an Protein S > 5 U/ml ist und der Gehalt an Faktor II, Faktor X und Faktor IX < 0,5 U/ml ist.

6. Verwendung des Konzentrats nach den Ansprüchen 2 bis 5 für die Herstellung pharmazeutischer Zubereitungen für die Behandlung von Sepsis sowie für angeborene Mängel an Protein C und Protein S.

7. Verwendung nach Anspruch 6, worin die Formulierungen in einer Form für eine parenterale Verabreichung vorliegen.

8. Verwendung nach Anspruch 6, worin die Formulierung aus einem Lyophilisat der Lösung, die Protein C und Protein S enthält, und WFI (Wasser für eine Injektion) für deren Rekonstitution besteht.

## Revendications

1. Procédé d'obtention d'un concentré de protéine C comprenant également la protéine S, dans lequel :
a) la fraction recueillie après le deuxième lavage de la résine DEAE Sepharose utilisée pour l'obtention du complexe de prothrombine purifiée (PTC) en commençant par du plasma humain congelé est dialysée avec un tampon approprié et est concentrée ;
b) le produit dérivé de l'étape précédente est soumis à la chromatographie sur une résine d'échange anionique faible, et le pH de l'éluat contenant les protéines concernées est ajusté, suivi par la dialyse avec le tampon de conditionnement de la résine utilisée dans la deuxième étape chromatographique ;
c) une deuxième étape chromatographique est exécutée sur une résine qui lie sélectivement la protéine C et la protéine S, où ladite résine est l'hydroxyapatite ;
d) le mélange de protéines purifié obtenu de cette manière est dialysé avec un tampon de formulation appropriée, ensuite préfiltré, nanofiltré et lyophilisé.

2. Concentré de protéine C comprenant également la protéine S obtenu par le procédé de la revendication 1.

3. Concentré selon la revendication 2, dans lequel la teneur en protéine S est égale à au moins 10% (calculé en U/ml) de la teneur en protéine C.

4. Concentré selon la revendication 3, dans lequel la teneur en facteur II, facteur X et facteur IX est <0,5 U/ml.

5. Concentré selon la revendication 4, dans lequel la teneur en protéine C est de 40 - 60 U/ml ; la teneur en protéine S est de >5U/ml et la teneur en facteur II, facteur X et facteur IX est <0,5 U/ml.

6. Utilisation du concentré selon les revendications 2 à 5 pour la préparation de compositions pharmaceutiques pour le traitement de la septicémie ainsi que pour des déficiences congénitales en protéine C et en protéine S.

7. Utilisation selon la revendication 6, où lesdites formulations se présentent sous une forme pour l'administration parentérale.

8. Utilisation selon la revendication 6, où ladite formulation consiste en un lyophilisat de la solution contenant la protéine C et la protéine S, et de WFI pour la reconstitution de celles-ci.
